# EUROPEAN PATENT SPECIFICATION

(11) **EP 2 654 890 B1**
(45) Date of publication and mention of the grant of the patent: **18.04.2018**
(21) Application number: 11811419.8
(22) Date of filing: 21.12.2011
(51) Int. Cl.: A61N 5/00, A61N 5/10, H05G 1/44

(54) **A MOBILE X-RAY UNIT**
MOBILE RÖNTGENEINHEIT
EQUIPEMENT RADIOLOGIQUE MOBILE

(30) Priority: 22.12.2010 NL 2005901; 23.12.2010 US 201061426917 P
(43) Date of publication of application: 30.10.2013
(73) Proprietor: Nucletron Operations B.V., 3905 TH Veenendaal (NL)
(72) Inventor: VAN DER VEEN, Johannes Simon, NL-3905 TH Veenendaal (NL); WOUDSTRA, Bas, NL-3905 TH Veenendaal (NL); HENNING, Johan, NL-3905 TH Veenendaal (NL)
(74) Representative: V.O.
(86) International application number: PCT/NL2011/050879
(87) International publication number: WO 2012/087132

(56) References cited:
- WO-A1-01/64286
- WO-A2-2007/064900
- US-A- 6 087 666
- US-A- 6 108 399
- US-A1- 2007 076 847
- US-B1- 6 241 670
- Topex, Inc: "Topexbrochure: SRT 100 Superficial Radiotherapy System for the Treatment of Skin Cancer", , 9 January 2007 (2007-01-09), XP002656846, Retrieved from the Internet: URL:http://www.rsllabin.com/pdf/TOPEXBROCH URE_v9.pdf [retrieved on 2011-08-15]
- Topex, Inc: "Regulatory Information", , 31 December 2007 (2007-12-31), XP002656847, Retrieved from the Internet: URL:http://www.topexmedical.com/product2.h tml [retrieved on 2011-08-15]
- Nancy C Brogdon: "5. 510(k) Summary", , 1 January 2004 (2004-01-01), XP055346297, Retrieved from the Internet: URL:https://www.accessdata.fda.gov/cdrh_do cs/pdf6/K063456.pdf [retrieved on 2017-02-15]
- John Brenna: "TOPEX and Therapy Remarketing Group Expand Distribution for Skin Cancer Treatment System", , 16 September 2008 (2008-09-16), Retrieved from the Internet: URL:http://www.prnewswire.com/news-release s/topex-and-therapy-remarketing-group-expa nd-distribution-for-skin-cancer-treatment- system-65116392.html [retrieved on 2017-02-15]
- Anonymous: "TOPEX", , 30 January 2008 (2008-01-30), XP055346502, Retrieved from the Internet: URL:http://web.archive.org/web/20080130220 208/http://www.topexmedical.com/product1.h tml [retrieved on 2017-02-16]

## Description

### FIELD OF THE INVENTION

The invention relates to a mobile X-ray unit comprising a base for accommodating a control unit and a power supply and further comprising an articulated displaceable arm supporting an X-ray applicator having an X-ray tube for emitting an X-ray beam through an exit window for irradiating an object.

The X-ray unit further comprises a built-in dosimetry system adapted to carry out real time dosimetry, wherein the dosimetry system is provided in the X-ray tube outside the path of the X-ray beam emitted from the anode target and passing through the exit window. The invention further relates to a method for dosimetry control of an X-ray beam emanating from the mobile X-ray unit.

### BACKGROUND OF THE INVENTION

Skin cancer, having increased incidence rate in the last decade of the 20^{th} century, requires substantial effort from medical professionals in terms of early diagnosis, logistics and availability of suitable treatment. However, it is appreciated that over 1.3 million new skin cancers are diagnosed annually and are increasing at a rate of about 5 % per year. Increased exposure to the sun without skin protection and a decreased ozone layer are regarded as the main causes of this increase - a problem estimated to be costing over 1 billion Euros in annual medical treatment expenses. Over 80% of skin cancers occur in the head and neck regions with 50% occurring in patients over 60 years of age. It is expected that a portion of the senior population will double in year 2025 compared to the present demographics.

Non proliferated cancers being substantially superficial lesions may be treated in different ways. First, surgery may be envisaged. However, such technique may be disadvantageous in terms of long waiting lists and complications related to post-treatment care. In addition, due to invasive character of surgery contamination of the wound by infections may present an additional risk. Secondly, irradiation using electrons of soft X-rays may be envisaged. Such techniques have an advantage of being non invasive, wherein a treatment session may be as short as 2 or 3 minutes. It will be appreciated that usually the integral treatment using a radiotherapeutic technique may comprise a number of sessions.

Accordingly, the growing incidence of skin cancer and increasing of a share of the senior population in overall demographics pose substantial challenge on the cancer treatment logistics.

Recently, the use of a portable X-ray unit has been suggested, which may be used inside a hospital radiotherapy department. An example of such portable unit is described in US 2007/0076851. The known unit comprises an X-ray applicator comprising X-ray source provided with a filtering device having a plurality of filters rotatably arranged with respect to a focal point of the X-ray tube for changing filtering characteristics on demand. The plurality of filters is arranged in a filtering device, which is transversely arranged with respect to a longitudinal axis of the X-ray tube. Such arrangement requires additional measure for delivering the X-ray beam towards the filter plane. The known device is used by positioning the X-ray applicator at some distance from the patient's skin.

It is a disadvantage of the known X-ray tube that poor control is available regarding actual delineation between the X-ray beam emanating from the X-ray applicator and a treated region on the patient.

WO 2007/064900 A2 by XOFT INC discloses an x-ray unit with built in real-time dosimetry. The dosimeter is attached outside of the unit. A smaller and less cumbersome unit would be desirable.

### SUMMARY OF THE INVENTION

The system and method of the invention are described in the independent claims.

It is an object of the invention to provide an improved mobile X-ray unit. More in particular, it is an object of the invention to provide the mobile X-ray unit wherein the X-ray beam may be delivered in a controlled way, as described in the claims. To this end the mobile X-ray unit according to the invention comprises a built-in dosimetry system adapted to carry out on-line or real time dosimetry.

It will be appreciated that the terms 'mobile' and 'portable' in the context of the present application may be interchanged as these terms equally relate to an easily moved or transported device, for example, a device which may be moved or transported by a single individual.

The dosimetry system is built-into the X-ray tube outside the path of the X-ray beam, according to the invention.

In other alternatives that are not part of the invention, the dosimetry system may be built into the X-ray applicator or the dosimetry system may be adapted to be arranged between the exit window of the X-ray applicator and the object while being connected to the controls of the mobile X-ray device.

It is found to be advantageous to provide a dosimetry system which may be adapted to deliver information on radiation dose distribution at or near the target area substantially in real time. The dosimetry system may comprise a film, a thermoluminescent device or a semiconductor detector. However, it will be appreciated that other types of commonly known dosimeters may be used as well. For example a suitable ionization chamber may be used, especially having a parallel plate configuration, like a Markus chamber, for example. It will be appreciated that for providing data on dose distribution at and/or near a target area, the dosimetry system may be adapted with a suitable plurality of dose detection devices, such as ionization chambers, thermoluminiscent devices, films, semiconductor detectors and so forth. This may be useful for controlling a profile of the X-ray beam.

When the dosimetry system is positioned inside the X-ray applicator it is preferably positioned outside a portion of the X-ray beam used for irradiating the patient. It will be appreciated that because the X-rays are generated substantially in three-dimensions, such placing of the dosimetry system is feasible.

In an example the dosimetry system is calibrated with respect to the absolute dose delivered by the X-ray tube. In this way reliable real time dosimetry may be carried out.

It will be appreciated that it is possible to either use a constant calibration value for converting the detector read-out signal into delivered dose, or, alternatively, to use a suitable equation, correcting for aging of the detector and/or for warming up of the X-ray tube. Still preferably, it is possible to use a calibration factor which maybe dependent on angulation of the X-ray applicator as changes in the internal alignment may cause a deviation of the delivered dose.

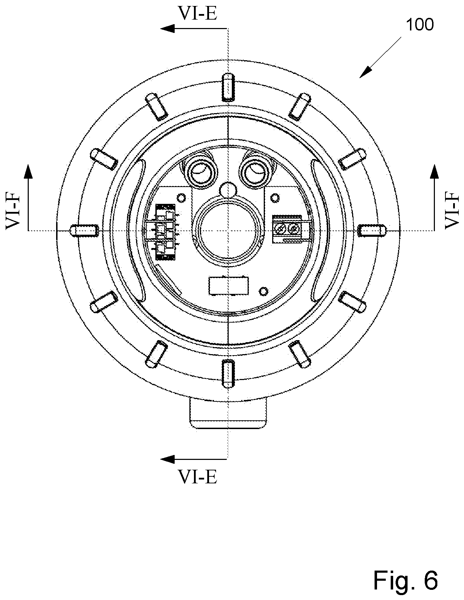

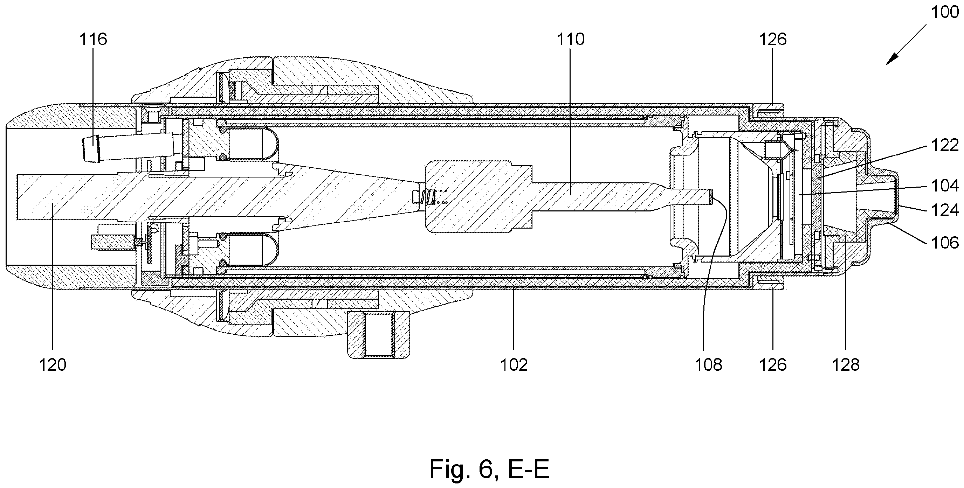

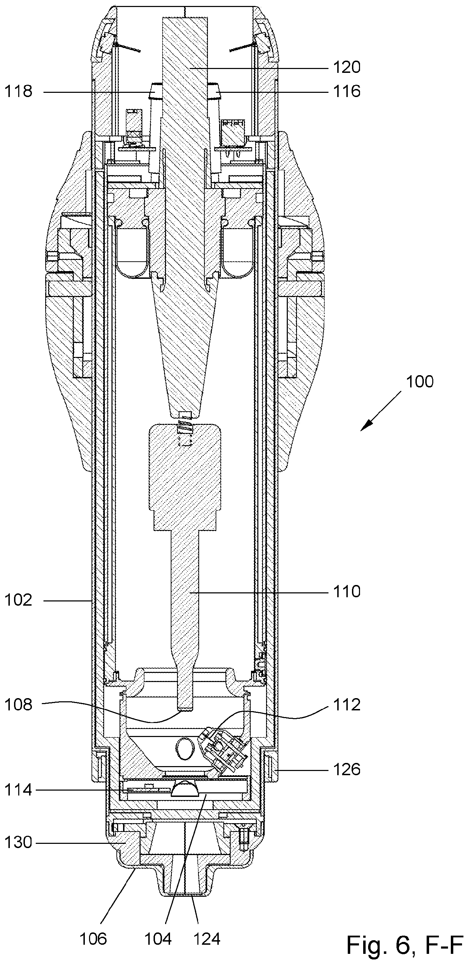

Preferably, the dosimetry system is adapted to provide a control signal to the main controls of the mobile X-ray upon switching on the X-ray tube. In addition, the dosimeter system may be adapted to provide a further control to the main controls of the mobile X-ray unit in the event the prescribed dose is delivered. More details on this example will be presented with reference to Figure 6.

Using a dosimetric device conceived to be positioned between the X-ray applicator and the object has additional advantages, as such device by virtue of its material ensures establishment of the electronic equilibrium at or near the surface of the object. As a result, the percentage depth dose build-up inside the object is more favorable with that of the prior art in terms of absolute value of the surface dose. It will be appreciated that for skin treatment the surface dose may not be higher than 137% of the prescribed depth dose. Usually the prescribed depth dose is specified at a depth of 5 mm from the skin surface.

Due to a presence of an additional material (a film or a detector) the percentage depth dose inside the object is favorably changed reducing the surface dose when normalized with the dose at 5 mm depth.

In an example of the X-ray unit, the dosimetry system comprises digital readout means. It is found to be particularly advantageous to enable a real-time data acquisition and data processing by using a digital dosimeter, which may be connected to the control unit of the mobile X-ray unit according to the invention for facilitating a substantially direct hard-ware response should the measured dose substantially deviate from the prescribed dose. It will be appreciated, that a film may be used for dosimetry purposes which may be subsequently read out using a digital densitometer.

It will be appreciated that the dosimetry system is preferably arranged to electronically communicate to the control unit, wherein the dosimetry system itself may have either an analogue or a digital signal as output. Those skilled in the art will readily appreciate which electronic devices (if any) may be necessary for enabling data communication between the dosimetry system and the control unit of the mobile X-ray unit.

In a still further example of the X-ray unit the dosimetry system is arranged to enable verification of at least a position and geometry of generated X-ray field.

The dosimetry system, i.e. a film or a suitable device (thermoluminiscent, ionization chamber or a semiconductor) may comprise a plurality of measuring points, preferably distributed in a plane. When such device is positioned in the X-ray field, the readings may be processed for establishing dose data across the applied field. For example, a reading at the central axis may be taken and a number of peripheral readings, preferably at different radial distances. As a result, information may be obtained regarding not only the absolute dose in the central field, but also information about beam flatness across the field. Preferably, the dosimetry unit is calibrated for enabling absolute dosimetry of deposited X-ray dose. Such calibration may be carried out using a phantom measurement for a known X-ray dose, for example.

In a still further example of the X-ray unit it further comprises an indicator for visualizing at least a portion of the X-ray beam emanating from the exit surface .

It is found that treatment efficacy is substantially improved when the indicator is provided for visually delineating at least a portion of the generated X-ray beam, like a central axis thereof, and/or full beam geometry.

In particular, such indication may be advantageous for positioning of the dosimetry system with respect to the X-ray beam. Preferably, the indicator comprises a light source. The light source may be arranged in the X-ray applicator or, alternatively it may be arranged around the outer surface of the X-ray applicator. In the former case the light indicator may be arranged to delineate the central axis of the X-ray beam and/or the full beam geometry, whereas in the latter case the light indicator may be arranged to delineate a central axis of the X-ray beam, preferably at a pre-determined distance from the X-ray applicator. Such feature may be advantageous when the X-ray applicator is used at a standard distance from the patient's skin. However, it will be appreciated that the light indicator arranged around the X-ray applicator may be adjustable for indicating the central axis of the X-ray beam at a variety of axial distances from the X-ray applicator.

In an example of the mobile X-ray unit, the indicator comprises an array of light sources concentrically arranged around the X-ray applicator. Although it may be sufficient to provide a single light source generating a narrow beam for indicating the central axis of the X-ray beam, it is found to be advantageous to provide a plurality of light sources generating respective narrow light beams intersecting at a given distance from an exit surface of the X-ray applicator. Due to this example, installation of the X-ray applicator at a prescribed distance from the skin is enabled as well as accurate installation of the dosimetry system with respect to the X-ray beam. In order to ensure a correct coverage of the target portion by the X-ray beam, the X-ray applicator may be positioned so that the indicated center of the X-ray beam is positioned substantially at a center of the target region. It will be appreciated that such an example functions particularly well for regular shaped X-ray beams, for example, when a circular, a square, an elliptic, or a triangular collimator is used for shaping the X-ray beam.

In a still further example of the mobile X-ray unit, the indicator comprises a light source accommodated inside the X-ray applicator for generating a light beam conceived to be intercepted by the collimator for providing a light image of the X-ray field emanating from the exit surface..

This arrangement is found to be particularly advantageous when the full shape of the X-ray beam is to be delineated, for example, in situations when an irregular beam shape is used. In such a case, preferably, the light source may be provided near the target or, via a mirror, off-axis, for generating a light beam conceived to be intercepted by the collimator. It will be appreciated that a direction of propagation of the light beam must be essentially conformal to a direction of propagation of the X-ray beam. In an example, when a mirror is used, the light source may advantageously be positioned off-axis.

In a still further example of the mobile X-ray unit the indicator comprises a light source and an optical fiber arranged to deliver light from the light source for interception by the collimator.

This example has an advantage that the light source may be positioned outside the X-ray applicator in order not to compromise its overall size. For example, the light source may be arranged in the base of the X-ray unit and the optical fibers may run from the base to inside the X-ray applicator for suitably illuminating the collimator for obtaining a light image equivalent to that of the generated X-ray beam.

In a still further example of the mobile X-ray unit the indicator may comprise a plurality of optical fibers distributed in the X-ray applicator in an area above the collimator for illuminating a collimator opening for causing the collimator opening to intercept the resulting light field. This arrangement may be advantageous for obtaining a light field having substantial intensity.

In a still further example of the mobile X-ray unit the indicator comprises a light source emitting a narrow light beam arranged inside the applicator for delineating the longitudinal axis of the X-ray beam. Preferably, a miniature laser source is used.

In a still further example of the X-ray unit a radiation detector is provided inside the outer housing for detecting the X-ray beam.

It is found to be advantageous to provide independent means for detecting presence of the generated X-ray beam. Preferably, the X-ray unit comprises a primary timer which sets a time for the high voltage supply for delivering a predetermined radiation dose. The radiation sensor accommodated inside the outer housing of the X-ray applicator may be part of a secondary timer circuit adapted to shut down the high voltage supply upon the predetermined radiation dose is delivered. In this way radiation safety control may be improved.

Preferably, in an example when the dosimetry system is operable to provide radiation dose data in real time, the signal from the dosimetry system may be used in addition to the signal from the built in radiation detector. In particular, when the dosimetry system is arranged to enable beam flatness verification, a substantial deviation from the prescribed beam flatness may be used as a control signal to shut-down the system.

In a still further example of the X-ray unit, the X-ray unit comprises an exit surface conceived to be directed towards a patient, said surface being covered by an applicator cap.

It is found advantageous to provide such an applicator cap as it may have several functions. First, the applicator cap may be used for protecting an exit window of the X-ray applicator from contamination. Secondly, thickness of the cap in a direction of the beam propagation may be selected to be sufficient for substantially eliminating electron contamination from the X-ray beam. Those skilled in the art will readily appreciate the relationship between the energy of the secondary electrons emanating from the X-ray tube and a required thickness of a given material, for example plastics, glass, ceramics sufficient for fully intercepting these electrons. Preferably, the applicator cap is disposable.

It will be appreciated that the indicator arranged for delineating the X-ray beam may be arranged with a sufficient intensity to provide a resulting field image through the applicator cap. Lasers are found to be particularly suited for this purpose. However, light emitting diodes may be used a well. Alternatively, an arrangement of one or more light sources generating a narrow beam outside the X-ray applicator may be advantageous as such one or more sources may be arranged on respective support arm such that the respective narrow light beams are not intercepted by the applicator cap.

According to the present invention there is provided a method for dosimetry control of an X-ray beam emanating from a mobile X-ray unit comprising a base for accommodating a control unit, a power supply and further comprising an articulated displaceable arm supporting an X-ray applicator having an X-ray tube comprising an anode target and a cathode and including a body having an exit window at one end thereof for generating an X-ray beam emitted from the anode target, the method comprising:
- measuring a radiation-related parameter associated with the X-ray beam using a built-in dosimetry system that is provided in the X-ray tube outside the path of the X-ray beam emitted from the anode target and passing through the exit window.

In a further example of the method, an indicator is provided in or near the X-ray applicator for visually delineating at least a portion of the X-ray beam for positioning the dosimetry system. Preferably, the indicator comprises a light source arranged to generate a light field conceived to be intercepted by a collimator opening for providing visualization of the X-ray beam. Alternatively, the indicator may comprise a light source arranged to delineate a longitudinal axis of the X-ray beam.

These and other aspects will be discussed with reference to drawings wherein like reference numerals or signs relate to like elements. It will be appreciated that the drawings are presented for illustration purposes only.

### BRIEF DESCRIPTION OF THE DRAWINGS

Figure 1a presents in a schematic way a mobile X-ray unit
Figure 1b presents in a schematic way a displaceable panel of the mobile X-ray unit.
Figure 1c presents in a schematic way a displacement functionality of the application in the X-ray unit.
Figure 2 presents in a schematic way an architecture of the mobile X-ray unit
Figure 3 presents in a schematic way a dosimetry system of the X-ray unit.
Figure 4a presents in a schematic way a first example of a cross section of an X-ray applicator of the mobile X-ray unit depicting a first example of the indicator.
Figure 4b presents in a schematic way a second example of a cross section of an X-ray applicator of the mobile X-ray unit depicting a second example of the indicator.
Figure 4c presents in a schematic way a third example of a cross section of an X-ray applicator of the mobile X-ray unit depicting a third example of the indicator.
Figure 5 presents in a schematic way an example of the X-ray applicator of Figure 3 provided with an applicator cap.
Figure 6 presents in a schematic way a further example of the X-ray tube of the X-ray mobile unit with a built-in dosimeter.

### DETAILED DESCRIPTION OF THE DRAWINGS

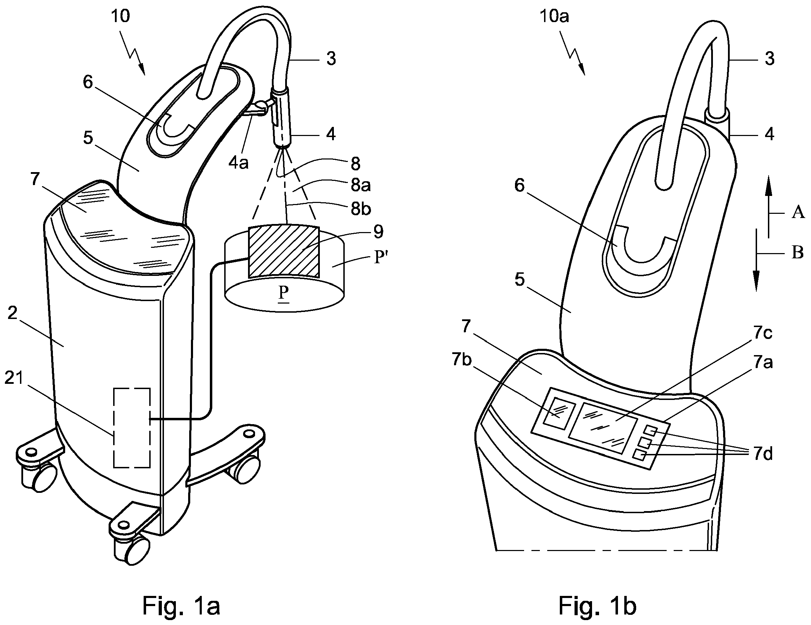

Figure 1a presents in a schematic way an example of a mobile X-ray unit. The mobile X-ray unit 10 comprises a base 2 comprising at least a power supply unit, a cooling system and a control unit for controlling an operation of the X-ray applicator 4 comprising an X-ray tube accommodated in an outer housing. The X-ray applicator 4 is connected with the base using flexible cables 3, which may be at least partially received in a displaceable panel 5. The applicator 4 is supported by an articulated displaceable arm 4a, which may comprise a pivot for altering angulation of the applicator 4 in space. The applicator 4 comprises a longitudinal axis and an exit window 8 through which the generated X-ray beam is emitted. The articulated arm 4a may also be mechanically connected with the displaceable panel 5 for enabling alteration of a vertical position of the applicator 4. Preferably, the displaceable panel 5 is provided with a handle 6 enabling easy manipulation thereof. The displaceable panel 5 may be guided along suitable rails for enabling a substantially smooth and shock-free displacement thereof.

Preferably, the X-ray applicator accommodating the X-ray tube has coaxial geometry, wherein the X-ray beam 8a conceived to irradiate a target region on a surface P' of a person P propagates from the exit window 8 having a beam axis 8b substantially corresponding to the longitudinal axis of the X-ray tube. This may be enabled by arranging an anode of the X-ray applicator so that a longitudinal axis of the anode is substantially parallel to the longitudinal axis of the X-ray applicator. A dosimetry system 9 is provided for providing data on at least a portion of the X-ray field 8a at or near the surface P' of the patient P. Preferably, for the dosimetry system a system capable of generating real-time data is selected. Ionization chambers and solid state detectors, for example semiconductor detectors are suitable for this purpose. Preferably, the signal from the dosimetry unit is supplied into the control unit 21 of the X-ray apparatus for the real-time dose delivery control and/or interrupt.

Preferably, for positioning the X-ray applicator 4 and the dosimetry system 9 with respect to the target region on the surface P' the applicator is provided with an indicator arranged to visually delineate the X-ray field to be generated by the X-ray tube inside the applicator 4. Preferably, the indicator comprises a light source, such as a light emitting diode, a laser or the like.

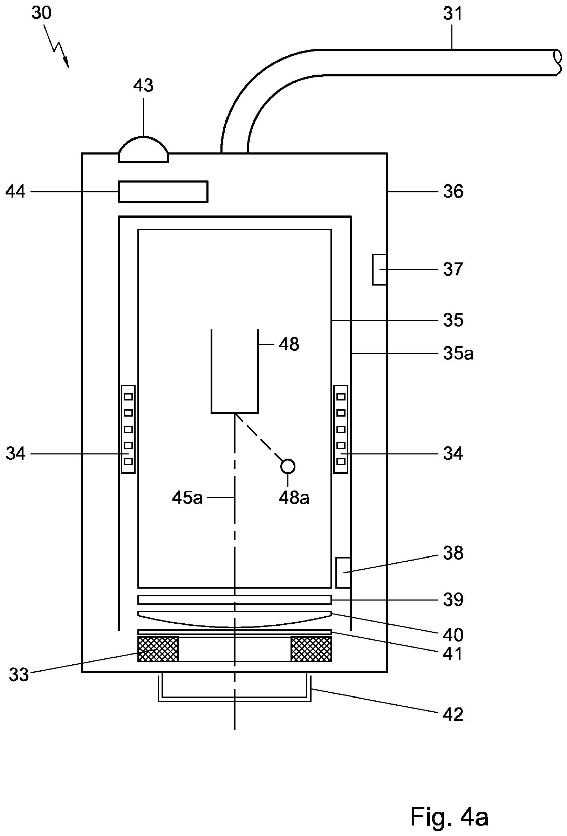

The light source may be arranged either inside the X-ray applicator 4, or around the X-ray applicator, or it may be remotely positioned, for example in the base 2. In the latter case light from the light from the light source (not shown) may be conducted towards the X-ray applicator using suitable one or more optical fibers. More details on the indicator will be presented with reference to Figures 4a - 4c.

Preferably, the X-ray unit 10 includes a base 2 supporting a displaceable panel 5 and housing a display 7 for feeding-back suitable user information. The display 7 may be arranged as a touch-sensitive screen for enabling suitable data input into the system. For example, the display panel may comprise mean for switching the light indicator on. Optionally, the light indicator may always be on when the X-ray unit is switched on. The user interface may further be used to input prescribed dose and, possibly, prescribed dose distribution, especially when dose modifiers are used for intruding a gradient in the dose profile across the X-ray field. The user interface may also be arranged to display data on actual dose delivery and dose distribution profile during the treatment. It will be appreciated that by using the dosimetry system the dose delivery protocol may be compared with actual dose delivery data in real time and, if necessary, the actual dose delivery may be corrected in real time and/or during further subsequent sessions should a discrepancy in prescribed and delivered dose of more than 1% occur.

Figure 1b presents in a schematic way an example of a displaceable panel 5 of the mobile X-ray unit. In this enlarged view 10a specific elements of the displaceable panel 5 are depicted. Accordingly, a handle 6 may be implemented as a mechanical item for pulling or pushing the panel 5. Alternatively, the handle 6 may be arranged as an electrical actuator for triggering motors (not shown) for displacing the panel 5. For example, when the handle 6 is pulled the motors may be activated for causing the panel 5 to displace in direction A. Pushing of the handle 6 may cause lowering of the panel 5 in direction B. Preferably, the mobile X-ray unit comprises means for limiting a travel distance of the panel 5. This may be advantageous for ensuring mechanical stability of the system on one hand (limitation of the upper level) and may be beneficial for preventing cable damage (limitation of the lower level). Preferably, the panel 5 is movable using built-in rails whose length may be chosen for limiting the displacement range of the panel 5 in a desirable way.

The display 7 may function as a suitable user interface 7a. For example, the patient data, such as a photo of the patient and/or a photo of a lesion may be provided in window 7b, whereby relevant patient information, such as the date of birth, gender, dose prescription and dose delivery protocol and so on may be displayed in window 7c. Buttons 7d may be provided as touch functionality for enabling entering data. Alternatively or additionally, suitable hardware switches or buttons may be provided as well.

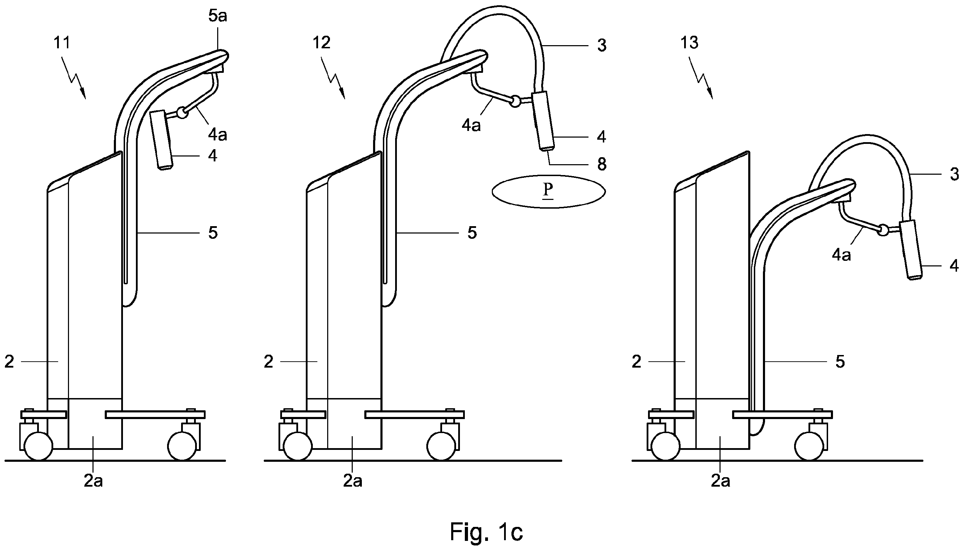

Figure 1c presents in a schematic way an example of displacement functionality of the application in the X-ray unit. The mechanical components of the mobile X-ray unit are developed and realized to support a broad range of translational and rotational movements for the X-ray applicator 4.

In view 11 of Fig. 1c a schematic example is presented wherein the X-ray applicator is in its parked position. It will be appreciated that cabling and optical fibers are not depicted for clarity reasons. Such position may be suitable for transport of the mobile X-ray unit towards a booth and/or for maneuvering the X-ray unit around the patient. In order to retract the X-ray applicator as close as possible to the base 2, the articulated arm 4a may be bent under the outer portion 5a of the displaceable panel 5. For ensuring stability of the mobile X-ray unit during maneuvering thereof, a load block 2a close to a floor is provided for lowering an absolute position of the center of gravity of the overall construction.

View 12 of Fig. 1c presents in a schematic way a further possibility, wherein the X-ray applicator 4 is in one of its working positions having an X-ray exit surface 8 being oriented towards a patient P. In order to suitably position the X-ray applicator with respect to the patient P, the displaceable panel may be moved to a certain dwell position located between the lowest position and the highest position of the panel 5. The articulated arm 4a may be used for suitably rotating the X-ray applicator about a rotation axis. Preferably, a rotation axis is selected to coincide with a direction of emanation of the X-ray beam from the exit surface when the X-ray tube is vertically oriented.

View 13 of Fig. 1c presents in a schematic way a still further possibility, wherein the X-ray applicator 4 is to be used at a lowered position. For this purpose the displaceable panel 5 may resume its lowest stand and the arm 4a may be used for orienting the X-ray applicator in a desirable way.

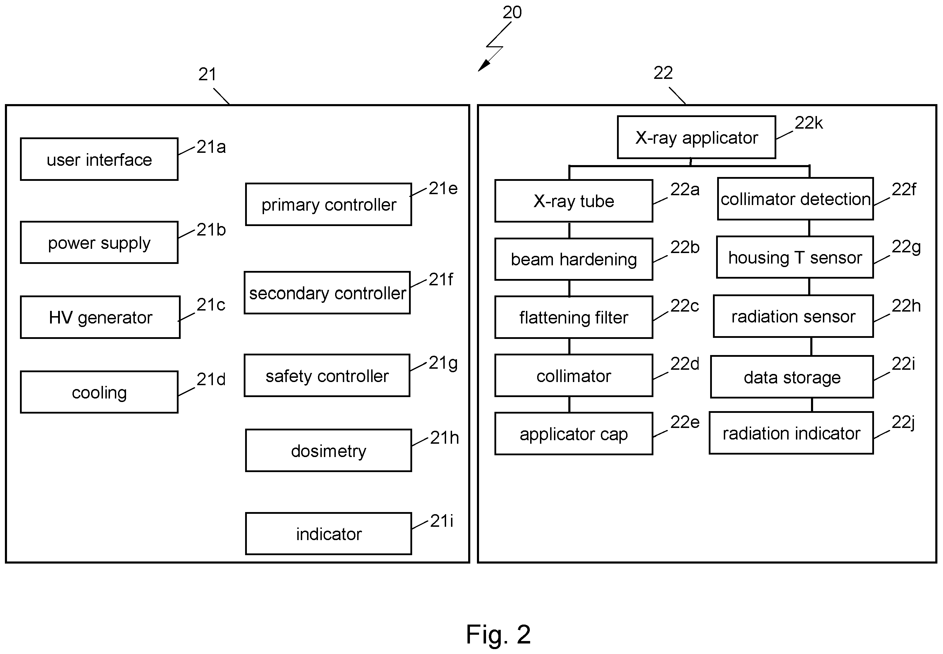

Figure 2 presents in a schematic way an example of architecture of the mobile X-ray unit. The mobile X-ray unit comprises a high voltage power supply, preferably adapted to generate 50 - 75 kV X-rays in a suitable X-ray tube, a cooling system for cooling the X-ray tube during use and a control system for controlling electronic and electric parameters of sub-units of the X-ray unit during use. View 20 schematically depicts main units of the control system 21 and of the X-ray applicator 22.

The control system 21 preferably comprises a hard wired user interface 21a for enabling switching on and switching off of the high voltage supply 21b. Preferably, the high voltage supply 21b comprises a high voltage generator 21c with improved ramp-up and ramp-down characteristics. Preferably, the ramp-up time is of the order of 100 ms. The hard wired interface 21a, may also be arranged to automatically switch on the cooling system 21d upon an event the high voltage generator is switched on. In addition, the control system 21 may comprise a primary controller 21e arranged for controlling the dose delivery of the X-ray applicator in use. Such primary controller 21e may be provided with a primary counter adapted to register time lapsed after the X-ray radiation is initiated. The primary counter may then automatically switch off the high voltage supply to the X-ray tube upon an event a pre-determined dose is reached. It will be appreciated that the pre-determined dose is at least dependent on the energy of generated X-rays and the dose rate, wherein such dependence may be calibrated in advance. Provided corresponding calibrated data is made available to the primary controller adequate primary dose delivery control may be achieved. Preferably, a secondary controller 21f is provided for enabling an independent loop of dose delivery control. The secondary controller may be connected to a dose meter accommodated inside the X-ray applicator in the X-ray field before the collimator. Accordingly, the dose meter may provide real-time data on actual dose delivery taking into account dose variation during ramp up and ramp down of the high voltage source. Still preferably, the control system may further comprise a safety controller 21g adapted to compare readings from the primary controller 21e and the secondary controller 21f for triggering switching off of the high voltage generator 21c wherein a desired dose is delivered. In addition or alternatively, the safety controller 21g may be wired to guard emergency stop, door interlock and a generator interlock.

The control system may further comprise a dosimetry control 21h, adapted to communicate with a dosimetry system, preferably on-line. However, it is also possible that the dosimetry control 21h may accept data from a scanned dosimetric field and update dose delivery data using such post-processing.

The dosimetry control 21h is preferably arranged to provide an interrupt signal, should the real-time dosimeter measure a substantial deviation between the prescribed dose and the measured dose. For example, the dosimetry control 21h may provide a suitable interrupt signal to the high voltage generator control 21c.

The control system may further comprise an indicator controller 21i for controlling the light source for delineating at least a portion of the X-ray beam. Although for simplicity the indicator controller 21i may be linked to a power supply unit 21b for switching on the light source once the system is on, it is preferable, that the light source is switched on demand. Accordingly, the indicator control may be arranged to provide electrical power to the light source when triggered by the user. The user may provide a suitable trigger signal by means of a user interface, or, for example, using a dedicated hardware switch.

The X-ray applicator 22 may preferably comprise the following features: an X-ray tube 22a, conceived to be housed in an outer housing (shielding) 22k. In accordance with the invention the X-ray tube is provided having coplanar target, collimator and the exit window geometry causing the generated X-ray beam to propagate substantially parallel to the longitudinal axis of the X-ray tube. Preferably, a target-collimator distance is about 4 - 10 cm, preferably about 5 to 6 cm. The X-ray applicator may further comprise a beam hardening filter 22b selected to intercept low-energy radiation and a beam flattening filter 22c, designed to intercept portions of X-ray radiation for generating a substantially flat beam profile near the exit surface of the X-ray applicator. Further, the X-ray applicator 22 may comprise one or more collimators arranged to define treatment beam geometry. Preferably a set of collimators is used, having diameters of, for example, 1, 1.5, 2, 2.5, 3, 3.5, 4, 4.5, 5 cm. It will be appreciated that although circular collimators are discussed, collimators of any shape, like square, elliptic or custom made collimators are possible. It is found to be advantageous to provide the X-ray applicator 22 with automatic collimator detection means 22f adapted to automatically signal which collimator is being used. Preferably, resistive sensing is used, wherein each collimator is provided with at least a couple of projections for bridging a resistive path provided in a collimator receptacle. The resulting electrical resistance of the receptacle constitutes a signal representative of a collimator being used. The X-ray applicator 22 still further preferably comprises a built-in temperature sensor adapted to signal temperature of the X-ray tube and/or the outer housing (shielding). The signal from the temperature sensor is received by the control system which carried out analysis thereof. Should the measured temperature be elevated beyond an allowable level, an alarm signal may be generated. Optionally, a shut-off signal to the high voltage generator may be provided. The X-ray applicator 22 further comprises a radiation sensor 22h arranged inside the outer housing 22k for detecting X-ray radiation which is actually being delivered by the X-ray tube. Preferably, for safety reasons the X-ray applicator 22 further comprises a non-volatile data storage 22i arranged for recording operational parameters at least of the X-ray tube. Further, to enhance radiation safety, the X-ray applicator 22 may be provided with a radiation indicator 22j arranged for providing a visual and/or an audio output to the user and/or the patient regarding ON/OFF condition of the X-ray tube. It will be appreciated that the radiation indicator 22j may comprise a plurality of distributed signaling means. Preferably, at least one signaling means, for example a light emitting diode (LED) is associated with the X-ray applicator 22. More preferably, the signaling means is provided on the X-ray applicator 22.

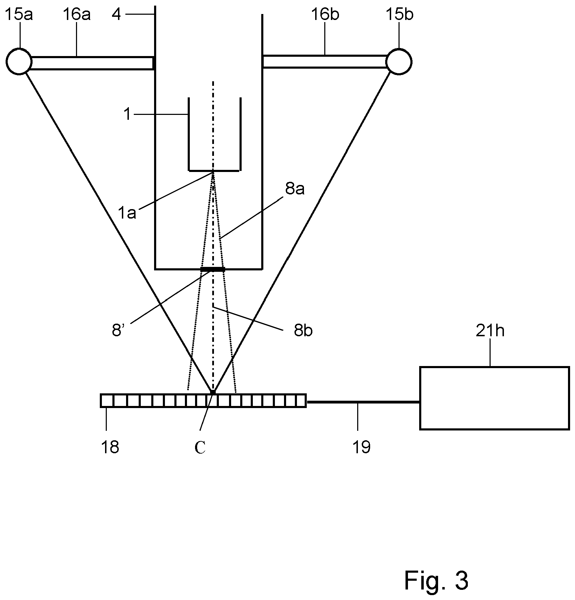

Figure 3 presents in a schematic way a dosimetry system of the X-ray unit. The X-ray applicator 4 discussed with reference to the foregoing, comprises an X-ray tube arranged with an anode 1 having a target region 1a for generating a diverging X-ray beam 8a. The target region 1a is a substantially flat plate which extends substantially perpendicular to the longitudinal axis of the anode 1. Although preferably the anode 1 is oriented coaxially with the axis 8b of the X-ray beam (and the X-ray tube), other respective orientations are possible. The generated X-ray beam is emitted by the X-ray applicator from an exit surface 8'. It will be appreciated that suitable filters, a collimator and an exit window of the X-ray tube are not depicted for clarity reasons. Accordingly, the exit surface 8' does not necessarily correspond to the exit window of the X-ray tube.

Preferably, for positioning the X-ray applicator 4 with respect to a target region of the patient, an indicator is used. The indicator may comprise two light sources 15a, 15b arranged to generate a narrow beam light, said light sources being mounted on respective support arms 16a, 16b and by their means to the outer surface of the X-ray applicator 4. Preferably, the light sources are arranged to provide a point in space C corresponding to the beam axis 8b. The dosimetry system 18 may then be centralized with respect to the point C for intercepting the X-ray beam.

The X-ray unit according to the invention may be provided with a plurality of dosimetric devices of different size. A suitable dosimetric device may be selected based on the actual beam size. Preferably, the dosimetric device 18 extends further than the X-ray field for measuring an absolute dimension of the delivered X-ray field.

Preferably, the dosimetry system 18 comprises an array of independent measuring volumes. It will be appreciated that for this purpose a film may be used, or a set of TLD devices or an array-type semiconductor dosimeter. As a result dose distribution across the X-ray field may be established for verification and/or for intra-fraction correction. Preferably, the dosimetric device provides real time reading, which may be provided using suitable cabling 19 to the dosimetry control unit 21h, as discussed with reference to Figure 2.

Although an example of the dosimetric system is discussed with reference to the X-ray applicator provided with the field delineation means, it will be appreciated that alternatively no indicator delineating the X-ray field may be provided.

Figure 4a presents in a schematic way a first example of a cross section of an X-ray applicator of the mobile X-ray unit depicting a first example of the indicator. The X-ray applicator 30 comprises an outer housing 36 accommodating the X-ray tube assembly 35 provided with external shielding 35a.

In accordance with an example, the X-ray applicator 30 further comprises a light source 48a cooperating with a mirror 48 for emitting a light a beam indicative of a beam of X-rays produced by the X-ray tube. Preferably, X-rays have a propagation axis 45a which coincides with a longitudinal axis of the X-ray tube. The light source 48a and the mirror 48 are arranged to cause the generated light beam to propagate substantially along the longitudinal axis of the X-ray tube assembly 45a.

When the thus formed light beam is intercepted by the collimator 33 a visual indication of the X-ray beam is enabled facilitating accurate alignment between the X-ray applicator and the target area of the patient.

Preferably, the distance between the target (anode) and the collimator 33 is in the range of 4 ... 10 cm, preferably about 5 to 6 cm. Such relatively short target-collimator distance is surprisingly suitable for generating an X-ray beam having a substantially narrow penumbra (1.5 - 1.8 mm for 20/80% lines) and good beam flatness due to a relatively small focal size.

The X-ray applicator 30 further comprises a filter 39 for hardening the X-ray beam emanating from the target, a beam flattening filter 40 for flattening out a beam profile and collimator 33 insertable in a collimator receptacle 41.

In order to prevent overheating of the X-ray tube in use a cooling system 34 is provided, which may advantageously be arranged in spacing between the X-ray tube 35 and the shielding 35a in contact with the surface of the X-ray tube 35. A suitable coolant may be provided using a pipe 31. Preferably, the coolant is circulating and may alternatively be water or pressurized gas. The X-ray applicator 30 may comprise a temperature sensor 37.

The X-ray assembly 30 may further comprise a suitable radiation detector 38, connected to a radiation indicator 43. Preferably, data collected by the radiation detector 38 is stored in a data storage unit 44. In order to protect an X-ray exit surface of the X-ray applicator 30 from intra-patient contamination, an applicator cap 42 may be provided to cover at least the exit window of the X-ray applicator 30. Preferably, the applicator cap is thick enough to fully intercept secondary electrons emanating from the X-ray applicator. Preferably, the applicator cap is manufactured from PVDF (polyvinylidene fluoride) and is about 0.4 - 0.7mm, preferably 0.6 mm thick across the window portion, having density of about 1.75 - 1.8, preferably 1.78. Alternatively the applicator cap may be 0.3 - 0.6mm, preferably 0.5 mm thick across the window portion and having density of 1.30 - 1.45, preferably 1.39, being manufactured from PPSU (polyphenylsulphone). It is found that these materials are particularly suitable as they as stable under influence of the X-rays and are suitable for different types of sterilization procedures, such as chemical sterilization, or sterilization under elevated temperatures.

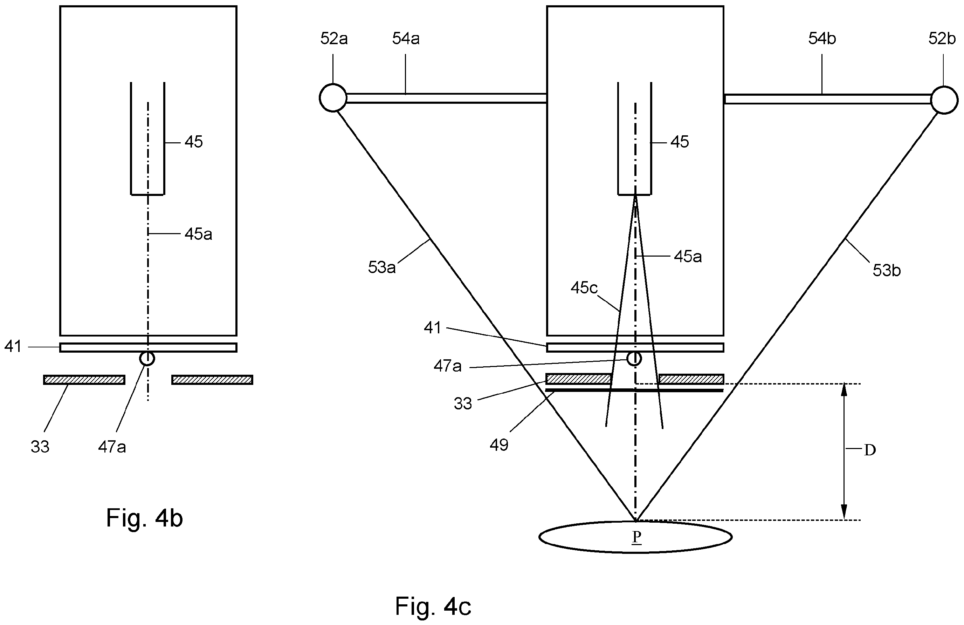

Figure 4b presents in a schematic way a second example of a cross section of an X-ray applicator of the mobile X-ray unit depicting a second example of the indicator. In this exemplary example an optical fiber 47a is provided in the collimator receptacle 41 above the collimator 33. The optical fiber 47a is arranged to generate a light field being substantially centered about the collimator opening 33 for simulating an X-ray beam emitted through the collimator. For this purpose the optical fiber 47a is arranged to emit a substantially narrow beam having divergence representative with expected divergence of the X-ray beam.

Alternatively, it is possible to use the optical fiber 47a for visualizing a central axis 45a of the X-ray beam. In this case the optical fiber is advantageously arranged to emit a narrow beam light producing a miniature light spot on a surface of the patient. Preferably, a dimension of the light spot is less than 5 mm², more preferably a dimension of the light spot is about 1 mm². A suitable light emitting diode or a laser may be used for generating light emanating from the fiber 47a. Preferably, the light emitting diode and the laser are remotely arranged with respect to the X-ray applicator 30. It will be appreciated that an alternative configuration may be used wherein one or more light sources cooperate with one or more optical fibers.

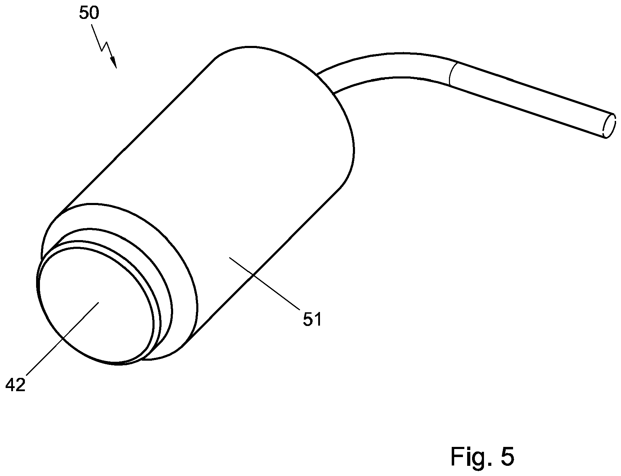

Figure 4c presents in a schematic way a third example of a cross section of an X-ray applicator of the mobile X-ray unit depicting a third example of the indicator. In this particular example the X-ray applicator having a target 45 for generating an X-ray beam 45c having the longitudinal X-ray axis 45a is provided with external indicator for visualizing the longitudinal axis 45a at a pre-determined distance D from the lower surface 49 of the X-ray applicator. It will be appreciated that the lower surface 49 may relate to the exit window as discussed with reference to Figure 1c, or it may relate to the applicator cap as is discussed with reference to Figure 5.

The external indicator comprises one or more light sources 52a, 52b disposed on respective support arms 54a, 54b for generating respective narrow light beams 53a, 53b said beam being directed towards the axis 45a and being adapted to intersect at the pre-determined distance D from the lower surface 49 of the X-ray applicator 30. Preferably, the Distance D is selected to be between 0.5 and 2 cm. The support arms 54a, 54b are arranged in such a way that the light beams 53a, 53b do not intercept the X-ray applicator.

When positioning the X-ray applicator with respect to the patient P, the former must be maneuvered in such a way that the beams 53a, 53b intersect at the surface of the patient. However, should the treatment regime suppose use of a dose build-up material, the beams 53a, 53b may cross on a surface of the dose build-up material. Preferably, the support arms 54a, 54b are adjustable for enabling indication of the central axis 45a at different distances from the lower surface 49 of the X-ray applicator.

In order to calibrate adjustment of the support arms, a transparent calibration phantom may be used, wherein the central axis and depth are marked. It will be appreciated that although Figures 4a - 4c disclose separate examples of the indicator, combinations of such embodiments is contemplated as well. For example, means for indicating the central axis may be combined with means for indicating the complete field. In addition, internal and external indicators may be combined as well.

Figure 5 presents in a schematic way an example of the X-ray applicator of Figure 3 provided with an applicator cap. The applicator cap 42 should be manufactured from a material that is transparent to X-rays, such as glass, plastics or ceramics. It is also possible, although not preferable to manufacture the applicator cap from a metal. In the latter case the applicator cap may be sterilized, however, it is preferably to use a disposable applicator cap. In view 50 of Figure 5 it is seen that the outer dimension of the X-ray applicator 51 may be larger that the outer dimension of the exit portion covered by the applicator cap 42. Although such an example is preferable for minimizing total weight of the X-ray applicator, it is possible that the exit portion has the same dimension as the body of the X-ray applicator 51. The applicator cap may be 0.5 - 2 cm thick when manufactured from a low Z material.

Figure 6 presents in a schematic way a further example of the X-ray tube of the X-ray mobile unit. The X-ray tube 100 has a body 102 enclosing at one end an end window 104 through which the X-rays pass. The end window is made from a thin sheet of Beryllium metal. Covering the end window 104 to provide protection against the damage of the window and protection against the toxic effects of the metal is an applicator cap 106. Applicator cap 106 is preferably made from a plastic material.

In the tube body 102 a target 108 is located at between 4 -10 cm from a collimator 130, and preferably at 4-6cm from the collimator 130, see Figure 6, cross-section F-F. The target is made from Tungsten metal to provide the desired X-ray spectrum. The tungsten tip of the target is mounted on a large anode assembly 110 which also serves to conduct away the heat created from the generation of the X-rays in the target. Most of the anode assembly is made from copper. The cathode 112 is located slightly off-axis near the end window. Electrons emitted from the cathode are accelerated across the gap by the potential difference between the cathode and anode, in this case set at about 70kV, to the target which they impact and cause the generation of X-rays in a known manner. X-rays emitted from the target 108 pass through a beam hardening filter 122 before passing through a collimator 130 and an exit surface 124 on an applicator cap 106. The collimator 130 may be housed in a suitable collimator receptacle 128.

The anode assembly 110 is mounted in the body 102 and electrically insulated from it. One of a number of known techniques and materials can be used to provide the desired level of insulation between the anode and the body 102.

As is also well known in the art, the production of X-rays generates large amounts of waste heat, with the result that it is necessary to cool the tube in order to maintain it at a safe temperature. Various cooling mechanisms are known and used in the art. In this example, the tube is cooled by means of water forced around the anode region. Water enters the back of the tube by means of conduits 116 and leaves by means of a second conduit 118. The water cooling circuit is a closed loop circuit, with the water leaving the tube assembly to be cooled by a remote cooler (not shown) before returning to the tube. Alternatively oil or another liquid could be used as the cooling medium. It is also known that a pressurized gas is used as an effective coolant in some applications.

As is known in the art, X-rays are generated and emitted in all directions, but the shielding by the body of the tube 102 and other internal components will tend to reduce the amount of radiation emitted from the body of the tube to a minimum, with most of the radiation emitted from the end window. The thickness of the shielding provided by the body will be such that it provides at least the minimum level of shielding required for safe use by the operator.

A high voltage cable assembly 120 is connected to the anode assembly 110. The high voltage cable assembly is connected to flexible cable means (not shown) which in turn is connected to a high voltage power supply. According to the invention, a radiation detector 114 forming the dosimetry system is placed outside the path of the X-ray beam emitted from the target 108 and passing through the end window 104. This detector can be any known form of radiation detector. It may be a known form of suitably radiation hardened semi-conductor connected to an amplifier. The radiation detector 114 detects when the tube 102 is working and emitting X-ray energy. Output from the detector is connected to a control unit, the output signals from which may be used to provide an optical indication of whether the tube is operating or not. By this means an X-ray detector is provided which can be used to detect whether the tube is on or off.

With further calibration of the radiation detector 114, it is possible to determine and calculate the X-ray dose administered to the patient during the treatment. By this means it is possible to have a real time dosimetry measurement system, in which the precise amount of radiation dose administered can be determined. Once the dose rate is known, a treatment plan can be modified during treatment. This is advantageous because it enables a very accurate and carefully controlled dose of X-rays to be administered.

In order to enable the tube 102 to be placed accurately over a tumour, a tumour illumination means is used. The tumour illumination means comprises a plurality of lights 126 placed around the circumference of the tube near the end window. When in use, the lights shine onto the skin of the patient. Since the lights 126 are positioned around the circumference of the tube body 102, at a short distance from the end of the tube they create a circle of light with a sharp cut off of the inner part of the circle. In this way, the position of the lights on the tube body 102 creates a shadow. This shadow circle is used to indicate the region which will be subject to irradiation when the X-ray tube is turned on. It should be appreciated the area within the circle will not be completely dark; the ambient light will be able to enter the shadow region.

Preferably the lights 126 are white LEDs which can be bright enough to clearly illuminate the target region but do not generate amounts of heat and have very long lives. The lack of heat generation is important because the lights will be in close proximity to the skin of the patient, and so it is important to minimise the risk of burning or other damage to the skin. Other colours of LEDs could be used. Alternatively, other light sources could be used, such as known filament lamps or even a remote light source connected to the ring by fibre optic cables.

While specific examples and embodiments have been described above, it will be appreciated that the invention may be practiced otherwise than as described. The descriptions above are intended to be illustrative examples. Thus, it will be apparent to one skilled in the art that modifications may be made to the examples. The scope of the invention is defined in the claims set out below.

## Claims

1. A mobile X-ray unit comprising a base for accommodating a control unit and a power supply, further comprising an articulated displaceable arm supporting an X-ray applicator having an X-ray tube (100, 102) comprising an anode target (108, 110) and a cathode (112) and including a body (102) having an exit window (104) at one end thereof for emitting an X-ray beam from the anode target through the exit window for irradiating an object, the X-ray unit further comprises a built-in dosimetry system (114) adapted to carry out real time dosimetry wherein the dosimetry system is provided in the X-ray tube outside the path of the X-ray beam emitted from the anode target and passing through the exit window

2. The mobile X-ray unit according to any preceding claim, wherein the dosimetry system is provided with digital readout means.

3. The mobile X-ray unit according to claim 2 wherein the dosimetry system is arranged to provide a signal to the control unit.

4. The mobile X-ray unit according to any one of the preceding claims, wherein the dosimetry system is arranged to enable verification of at least a position and geometry of a generated X-ray field.

5. The mobile X-ray unit according to any one of the preceding claims, wherein the dosimetry system is calibrated for enabling the absolute dosimetry of a deposited X-ray dose.

6. The mobile X-ray unit according to any one of the preceding claims, wherein the X-ray unit further comprises an indicator (126) for providing a visual indication of at least a portion of the X-ray beam emitted through the exit window.

7. The mobile X-ray unit according to claim 6, wherein the indicator comprises a light source.

8. The mobile X-ray unit according to claim 7 wherein the light source is a light emitting diode (LED) or a laser.

9. The mobile X-ray unit according to claim 1, wherein the dosimetry system is arranged to generate a further control signal upon generation of the X-ray beam.

10. The mobile X-ray unit according claim 6, wherein the dosimetry is calibrated for correcting for a parameter selected from a group consisting of: the temperature of the X-ray tube, the age of the X-ray tube, angulation of the X-ray tube, the energy of the X-ray beam.

11. The mobile X-ray unit according to any one of the preceding claims, wherein the dosimetry system is adapted to deliver information about radiation dose distribution at and/or near a target area.

12. A method for dosimetry control of an X-ray beam emanating from a mobile X-ray unit comprising a base for accommodating a control unit, a power supply and further comprising an articulated displaceable arm supporting an X-ray applicator having an X-ray tube comprising an anode target and a cathode and including a body having an exit window at one end thereof for generating an X-ray beam emitted from the anode target through the exit window for irradiating an object, the method comprising:
- measuring a radiation-related parameter associated with the X-ray beam using a built-in dosimetry system that is provided in the X-ray tube outside the path of the X-ray beam emitted from the anode target and passing through the exit window.

13. The method according to claim 12 further comprising the step of using an indicator for visually delineating at least a portion of the X-ray beam with respect to an object.

## Patentansprüche

1. Mobile Röntgeneinheit, umfassend eine Basis zum Aufnehmen einer Steuereinheit und einer Stromversorgung, ferner umfassend einen angelenkten verschiebbaren Arm, stützend einen Röntgenapplikator mit einer Röntgenröhre (100, 102), umfassend ein Anodenziel (108, 110) und eine Kathode (112), und einschließlich eines Körpers (102) mit einem Ausgangsfenster (104) an einem Ende davon zum Abgeben eines Röntgenstrahls von dem Anodenziel durch das Ausgangsfenster zum Bestrahlen eines Objekts, die Röntgeneinheit ferner umfassend ein eingebautes Dosimetriesystem (114), angepasst zum Ausführen von Echtzeitdosimetrie, wobei das Dosimetriesystem in der Röntgenröhre außerhalb des Wegs des Röntgenstrahls, abgegeben von dem Anodenziel und passierend durch das Ausgangsfenster, bereitgestellt ist.

2. Mobile Röntgeneinheit nach Anspruch 1, wobei das Dosimetriesystem mit digitalen Ablesemitteln versehen ist.

3. Mobile Röntgeneinheit nach Anspruch 2, wobei das Dosimetriesystem angeordnet ist, ein Signal für die Steuereinheit bereitzustellen.

4. Mobile Röntgeneinheit nach einem der vorhergehenden Ansprüche, wobei das Dosimetriesystem angeordnet ist, die Überprüfung von mindestens einer Position und Geometrie eines erzeugten Röntgenfeldes zu ermöglichen.

5. Mobile Röntgeneinheit nach einem der vorhergehenden Ansprüche, wobei das Dosimetriesystem kalibriert ist, um die absolute Dosimetrie einer hinterlegten Röntgendosis zu ermöglichen.

6. Mobile Röntgeneinheit nach einem der vorhergehenden Ansprüche, wobei die Röntgeneinheit ferner eine Anzeige (126) umfasst, um eine visuelle Anzeige von mindestens einem Teil des Röntgenstrahls, abgegeben durch das Ausgangsfenster, bereitzustellen.

7. Mobile Röntgeneinheit nach Anspruch 6, wobei die Anzeige eine Lichtquelle umfasst.

8. Mobile Röntgeneinheit nach Anspruch 7, wobei die Lichtquelle eine lichtemittierende Diode (LED) oder ein Laser ist.

9. Mobile Röntgeneinheit nach Anspruch 1, wobei das Dosimetriesystem angeordnet ist, ein weiteres Steuersignal beim Erzeugen des Röntgenstrahls zu erzeugen.

10. Mobile Röntgeneinheit nach Anspruch 6, wobei die Dosimetrie kalibriert ist zur Korrektur für einen Parameter, ausgewählt aus der Gruppe, bestehend aus: der Temperatur der Röntgenröhre, dem Alter der Röntgenröhre, der Anwinkelung der Röntgenröhre, der Energie der Röntgenröhre.

11. Mobile Röntgeneinheit nach einem der vorhergehenden Ansprüche, wobei das Dosimetriesystem angepasst ist, Information über Strahlungsdosisverteilung an und/oder nahe dem Zielbereich zu liefern.

12. Verfahren zur Dosimetriesteuerung eines Röntgenstrahls, ausgehend von einer mobilen Röntgeneinheit, umfassend eine Basis zum Aufnehmen einer Steuereinheit, einer Stromversorgung und ferner umfassend einen angelenkten verschiebbaren Arm, stützend einen Röntgenapplikator mit einer Röntgenröhre, umfassend ein Anodenziel und eine Kathode, und einschließlich eines Körpers mit einem Ausgangsfenster an einem Ende davon zum Erzeugen eines Röntgenstrahls, abgegeben von dem Anodenziel durch das Ausgangsfenster, zum Bestrahlen eines Objekts, das Verfahren umfassend: das Messen eines strahlungsbezogenen Parameters, verbunden mit dem Röntgenstrahl, unter Verwendung eines eingebauten Dosimetriesystems, das in der Röntgenröhre außerhalb des Wegs des Röntgenstrahls, abgegeben von dem Anodenziel und passierend durch das Ausgangsfenster, bereitgestellt ist.

13. Verfahren nach Anspruch 12, ferner umfassend den Schritt der Verwendung einer Anzeige zur visuellen Darstellung von mindestens einem Teil des Röntgenstrahls in Bezug auf ein Objekt.

## Revendications

1. Unité à rayons X mobile comprenant une base pour recevoir une unité de commande et une unité d'alimentation, comprenant en outre un bras déplaçable articulé supportant un dispositif d'application de rayons X ayant un tube de rayons X (100, 102) comprenant une cible d'anode (108, 110) et une cathode (112) et comprenant un corps (102) ayant une fenêtre de sortie (104) à une extrémité de celle-ci pour émettre un faisceau de rayons X depuis la cible d'anode à travers la fenêtre de sortie pour irradier un objet, l'unité à rayons X comprenant en outre un système de dosimétrie intégré (114) adapté pour effectuer une dosimétrie en temps réel dans laquelle le système de dosimétrie est agencé dans le tube de rayons X à l'extérieur du trajet du faisceau de rayons X émis par la cible d'anode et passant à travers la fenêtre de sortie.

2. Unité à rayons X mobile selon l'une quelconque des revendications précédentes, dans laquelle le système de dosimétrie est muni de moyens de lecture numériques.

3. Unité à rayons X mobile selon la revendication 2, dans laquelle le système de dosimétrie est agencé pour fournir un signal à l'unité de commande.

4. Unité à rayons X mobile selon l'une quelconque des revendications précédentes, dans laquelle le système de dosimétrie est agencé pour permettre une vérification d'au moins une position et une géométrie d'un champ de rayons X généré.

5. Unité à rayons X mobile selon l'une quelconque des revendications précédentes, dans laquelle le système de dosimétrie est calibré pour permettre la dosimétrie absolue d'une dose de rayons X déposée.

6. Unité à rayons X mobile selon l'une quelconque des revendications précédentes, dans laquelle l'unité de rayons X comprend en outre un indicateur (126) pour fournir une indication visuelle d'au moins une partie du faisceau de rayons X émis à travers la fenêtre de sortie.

7. Unité à rayons X mobile selon la revendication 6, dans laquelle l'indicateur comprend une source de lumière.

8. Unité à rayons X mobile selon la revendication 7, dans laquelle la source de lumière est une diode électroluminescente (DEL) ou un laser.

9. Unité à rayons X mobile selon la revendication 1, dans laquelle le système de dosimétrie est agencé pour générer un signal de commande supplémentaire lors de la génération du faisceau de rayons X.

10. Unité à rayons X mobile selon la revendication 6, dans laquelle la dosimétrie est calibrée pour corriger un paramètre sélectionné dans un groupe comprenant : la température du tube de rayons X, l'âge du tube de rayons X, l'angulation du tube de rayons X, l'énergie du faisceau de rayons X.

11. Unité à rayons X mobile selon l'une quelconque des revendications précédentes, dans laquelle le système de dosimétrie est adapté pour délivrer des informations concernant une distribution de dose de rayonnement au niveau et/ou à proximité d'une zone cible.

12. Méthode pour une commande de dosimétrie d'un faisceau de rayons X émanant d'une unité à rayons X mobile comprenant une base pour recevoir une unité de commande, une alimentation électrique, et comprenant en outre un bras déplaçable articulé supportant un dispositif d'application de rayons X ayant un tube de rayons X comprenant une cible d'anode et une cathode et comprenant un corps ayant une fenêtre de sortie à une extrémité de celle-ci pour générer un faisceau de rayons X émis depuis la cible d'anode à travers la fenêtre de sortie afin d'irradier un objet, la méthode comprenant :
- la mesure d'un paramètre en rapport avec un rayonnement associé au faisceau de rayons X en utilisant un système de dosimétrie intégré qui est agencé dans le tube de rayons X à l'extérieur du trajet du faisceau de rayons X émis par la cible d'anode et passant à travers la fenêtre de sortie.

13. Méthode selon la revendication 12, comprenant en outre l'étape consistant à utiliser un indicateur pour délimiter visuellement au moins une partie du faisceau de rayons X par rapport à un objet.
